# EUROPÄISCHE PATENTSCHRIFT

(11) **EP 0 563 502 B1**
(45) Veröffentlichungstag und Bekanntmachung des Hinweises auf die Patenterteilung: **29.11.1995**
(21) Anmeldenummer: 92810253.2
(22) Anmeldetag: 03.04.1992
(51) Int. Cl.: A61F 2/42, A61F 2/30

(54) **Metallschaft**
Metal stem
Tige en métal

(43) Veröffentlichungstag der Anmeldung: 06.10.1993
(73) Patentinhaber: SULZER Medizinaltechnik AG, 8404 Winterthur (CH); PROTEK AG, 3110 Münsingen-Bern (CH)
(72) Erfinder: Meuli, Hans Christoph, Prof. Dr. med., Bremgartenstrasse 117, CH-3012 Bern (CH); Koller, Hansjörg, CH-8400 Winterthur (CH)
(74) Vertreter: Hammer, Bruno, Dr.

(56) Entgegenhaltungen:
- EP-A- 0 115 564
- EP-A- 0 280 424
- DE-A- 2 309 432
- US-A- 4 725 280

## Beschreibung

Die Erfindung betrifft einen Metallschaft mit einem rechteckigen Kernquerschnitt für ein Implantat in einem Röhrenknochen, insbesondere für Kleingelenke, wie z.B. Fingergelenke.

Besonders für Implantate in Röhrenknochen von Kleingelenken ist es häufig erforderlich für die Verankerung, insbesondere für die Primärverankerung, eine Vielzahl unterschiedlicher Schaftgrössen und -Formen bereit zu halten, um den individuellen Gegebenheiten des einzelnen Patienten optimal entsprechen zu können und dadurch, vor allem während der ersten Zeit nach der Implantation, einen festen Sitz des Schaftes zu gewährleisten. Dies erfordert bisher für jedes Kleingelenk ein grosses "Sortiment" nur wenig voneinander verschiedener Schäfte; da bei Kleingelenken der eigentliche Gelenkteil meist untrennbar mit dem Schaft verbunden ist, sind bei den bekannten Implantat-Konstruktionen daher sogar für jeden Fall eine Vielzahl von Gelenkprothesen bereitzustellen.

Der Erfindung liegt die Aufgabe zugrunde, den geschilderten erheblichen Aufwand zu reduzieren und einen Schaft, insbesondere für Kleingelenke, zu schaffen, der, in einem gewissen Bereich, an verschiedene Knochenformen und -Grössen unterschiedlicher Patienten intraoperativ anpassbar ist. Die Lösung dieser Aufgabe erfolgt, dadurch, dass der Schaft zu seinem freien Ende hin an den Ecken des Kernes vorstehende, plastisch verformbare Flügel aus Blech aufweist, die durch eine Anzahl mindestens annähernd senkrecht zur Schaftachse verlaufender Einschnitte in Achsrichtung in mehrere Abschnitte unterteilt sind.

Die in Achsrichtung unterteilten, plastisch verformbaren Flügel ermöglichen dem Operateur, die Stellung ihrer einzelnen Abschnitte relativ zum Schaftkern während der Operation zu verändern, beispielsweise mit einer, an die Höhe ihrer Abschnitte angepassten, schmalen Flachzange zu verbiegen und so in einem gegebenen Knochenhohlraum, beispielsweise durch Klemmen zu verankern. Die Veränderung der Verankerungsform wird ohne die Erzeugung von Spänen intraoperativ vorgenommen.

Eine Vereinfachung bei der Herstellung des neuen Schaftes ergibt sich, wenn die Flügel für je zwei Ecken des Querschnittes zu einem wannenförmigen Blech verbunden sind, das seinerseits auf einer Seitenfläche des Kernquerschnittes befestigt ist. Weiterhin ist es für das intraoperative Anpassen der Flügelabschnitte zweckmässig, wenn die Höhe der Flügelabschnitte in Achsrichtung konstant ist. Ein Einsetzen des Schaftes in einen sich verjüngenden Knochenhohlraum wird erleichtert, wenn der Abstand der Flügelkanten vom Kern sich von distal nach proximal stetig vergrössert. Die zueinander unterschiedlich ausgebogenen Flügel werden beim Eintreiben elastisch verspannt und graben sich im Knochengewebe ein.

Bevorzugte Materialien für den neuen Schaft und die Flügel sind Titan und Titanlegierungen. Die zur Schaftachse senkrechten Einschnitte in den Flügeln können beispielsweise durch Wasserstrahlschneiden, Drahtschneiden mit Hilfe von Funkenerosion oder durch Stanzen hergestellt werden. Weiterhin erfolgt die Befestigung der Bleche auf dem Kern am einfachsten durch Schweissen, wobei die Wannenform vor oder nach dem Schweissen erzeugt werden kann.

Im folgenden wird die Erfindung anhand eines Ausführungsbeispiels im Zusammenhang mit der Zeichnung näher erläutert.
- Fig. 1: zeigt in räumlicher Darstellung eine Ansicht des neuen Schaftes;
- Fig. 2: ist ein Schnitt II-II von Fig. 1;
- Fig. 3: gibt in gleicher Darstellung wie Fig. 1 den intraoperativ "veränderten" Schaft wieder.

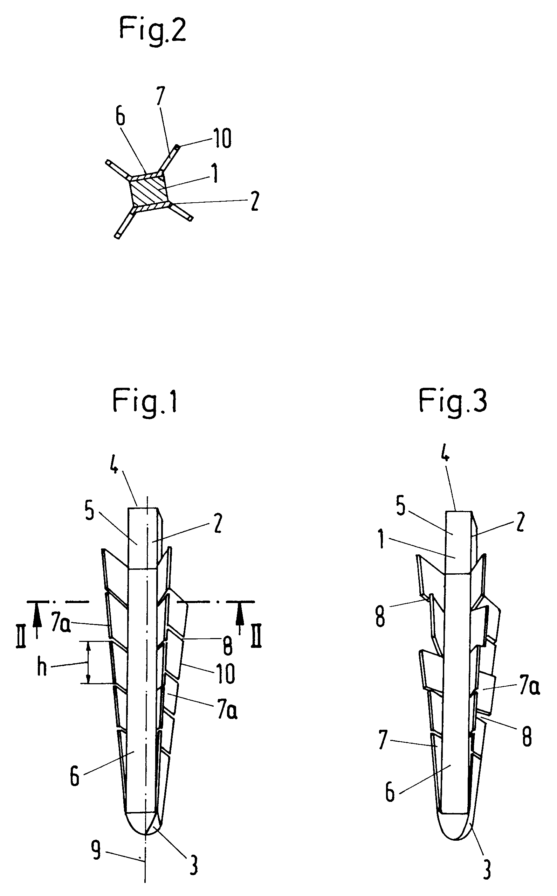

Der Schaftkern 1 ist in seinem Querschnitt rechteckig (Fig. 2) oder quadratisch und hat über seine ganze Höhe konstante Seitenlängen. Distal laufen seine vier Kanten 2 zu einem abgerundeten Endstück 3 zusammen. Am proximalen Ende 4 des vierkantigen Kerns 1 sitzt der eigentliche Gelenkteil der Prothese, der in dem gezeigten Beispiel nicht dargestellt ist.

Auf zwei einander gegenüber liegenden Seiten 5 des Kernes 1 ist je ein wannenförmig abgewinkeltes Blech 6 befestigt, z.B. aufgeschweisst.

Die beiden abgewinkelten Endstücke der Bleche 6 bilden plastisch verformbare Flügel 7, die bei Auslieferung der Schäfte mindestens im wesentlichen in Richtung der Diagonalen des rechteckigen Kernquerschnittes ausgerichtet sind. Durch eine Anzahl über die Höhe des Schaftes im Abstand voneinander angeordneter Einschnitte 8 sind die Flügel 7 in Richtung der Schaftachse 9 in mehrere Abschnitte 7a unterteilt, die während der Operation einzeln, beispielsweise durch Biegen mit einer Flachzange verformt werden können - wie in Fig. 3 verdeutlicht. Die Einschnitte 8 verlaufen mindestens annähernd in Ebenen senkrecht zur Schaftachse 9, wobei im gezeigten Beispiel die Einschnitte 8 für alle vier Flügel 7 jeweils auf gleicher Höhe liegen und ihre Abstände h in Achsrichtung ebenfalls gleich sind.

Um das Einsetzen des Schaftes in den operativ vorbereiteten Knochenhohlraum zu erleichtern, nehmen die Abstände der Flügelkanten 10 von den Kanten 4 des Kernquerschnittes in Richtung distal/proximal stetig zu.

## Patentansprüche

1. Metallschaft mit einem rechteckigen oder quadratischen Kernquerschnitt für ein Implantat in einem Röhrenknochen, insbesondere für Kleingelenke, dadurch gekennzeichnet, dass der Schaft zu seinem freien Ende hin an den Ecken (2) des Kernes (1) vorstehende, plastisch verformbare Flügel (7) aus Blech aufweist, die durch eine Anzahl mindestens annähernd senkrecht zur Schaftachse (9) verlaufende Einschnitte (8) in Achsrichtung in mehrere Abschnitte (7a) unterteilt sind.

2. Schaft nach Anspruch 1, dadurch gekennzeichnet, dass die Flügel (7) für je zwei Ecken (2) des Querschnittes zu einem wannenförmigen Blech verbunden sind, das seinerseits auf einer Seitenfläche (5) des Kernquerschnittes befestigt ist.

3. Schaft nach Anspruch 1 oder 2, dadurch gekennzeichnet, dass die Höhe (h) der Flügelabschnitte (7a) in Achsrichtung konstant ist.

4. Schaft nach einem der Ansprüche 1 bis 3, dadurch gekennzeichnet, dass der Abstand der Flügelkanten (10) vom Kern (1) sich von distal nach proximal stetig vergrössert.

## Claims

1. A metal shank having a core of rectangular or square cross-section for an implant in a hollow bone, especially for small joints, characterized in that the shank towards the free end of it exhibits plastically deformable lobes of sheetmetal which project at the corners (2) of the core (1) and in the axial direction are subdivided into a number of sections (7a) by a number of cuts (8) running at least approximately perpendicular to the axis (9) of the shank.

2. A shank as in Claim 1, characterized in that for every two corners (2) of the cross-section the lobes (7) are combined into a tub-shaped piece of sheetmetal which in turn is fastened to one sideface (5) of the cross-section of the core.

3. A shank as in Claim 1 or 2, characterized in that in the axial direction the height (h) of the sections (7a) of lobe is constant.

4. A shank as in one of the Claims 1 to 3, characterized in that the distance of the edges (10) of the lobes from the core (1) increases steadily from distal to proximal.

## Revendications

1. Tige métallique avec une section transversale rectangulaire ou carrée de l'âme pour un implant dans un os tubulaire, en particulier pour les petites articulations, caractérisée en ce que la tige présente, vers son extrémité libre, des ailettes (7) faites en tôle, plastiquement déformables, faisant saillie aux angles (2) de l'âme (1), qui sont partagées dans la direction axiale, en plusieurs portions (7a), par un certain nombre d'entailles (8) au moins à peu près perpendiculaires à l'axe (9) de la tige.

2. Tige selon la revendication 1, caractérisée en ce que les ailettes (7) sont reliées pour deux angles (2) de la section transversale en une tôle en forme de cuvette, qui de son côté est fixée sur une face latérale (5) de la section transversale de l'âme.

3. Tige selon la revendication 1 ou 2, caractérisée en ce que la hauteur (h) des portions (7a) des ailettes est constante dans la direction axiale.

4. Tige selon l'une des revendications 1 à 3, caractérisée en ce que la distance des bords (10) des ailettes par rapport à l'âme (1) augmente constamment du côté distal au côté proximal.
